# EUROPEAN PATENT SPECIFICATION

(11) **EP 2 677 937 B1**
(45) Date of publication and mention of the grant of the patent: **08.04.2020**
(21) Application number: 12707136.3
(22) Date of filing: 13.02.2012
(51) Int. Cl.: A61B 90/00, A61B 6/12

(54) **NON-RIGID-BODY MORPHING OF VESSEL IMAGE USING INTRAVASCULAR DEVICE SHAPE**
FORMUNG EINES GEFÄSSBILDES EINES NICHT STEIFEN KÖRPERS MITTELS EINER INTRAVASKULÄREN FORMVORRICHTUNG
MORPHAGE DE CORPS NON RIGIDE D'UNE IMAGE VASCULAIRE À L'AIDE D'UNE FORME DE DISPOSITIF INTRAVASCULAIRE

(30) Priority: 24.02.2011 US 201161446105 P
(43) Date of publication of application: 01.01.2014
(73) Proprietor: Koninklijke Philips N.V., 5656 AG Eindhoven (NL)
(72) Inventor: BARLEY, Maya Ella, 5656 AE Eindhoven (NL); DESJARDINS, Adrien Emmanuel, 5656 AE Eindhoven (NL); CHAN, Raymond, 5656 AE Eindhoven (NL); T'HOOFT, Gert Wim, 5656 AE Eindhoven (NL)
(74) Representative: Philips Intellectual Property & Standards
(86) International application number: PCT/IB2012/050623
(87) International publication number: WO 2012/114224

(56) References cited:
- WO-A1-2009/144730
- WO-A1-2009/153723
- WO-A1-2010/150147
- US-A1- 2005 182 319
- US-A1- 2006 058 643
- US-A1- 2009 088 629
- US-A1- 2009 137 952
- US-A1- 2009 216 114
- US-A1- 2010 030 063
- US-A1- 2010 169 815
- US-B1- 6 317 621

## Description

This disclosure relates to image registration, and more particularly to dynamic overlay morphing in accordance with dynamic behavior of internal organs, endoluminal anatomy, or vascular structures due to interventional medical devices.

Stand-alone fluoroscopy is a standard imaging modality for many interventional procedures. One significant drawback to x-ray usage is the lack of soft-tissue definition. While interventional devices are clearly visible, the treatment site (typically a soft tissue structure) is almost invisible unless some form of x-ray contrast agent is used to define it more clearly. Furthermore, such contrast agents are frequently nephrotoxic, and their use needs to be minimized. As a result, three-dimensional (3D) image overlay on live fluoroscopy would be desirable in many x-ray guided interventional procedures. The overlay would assist in the guidance of an interventional device to the treatment site by providing continuously-visualized, static, morphological information. The 3D overlay must accurately reflect the real anatomy (to within a few mm) to be clinically relevant, which is often a challenging task.

The 3D overlaid image may be either an intra-procedurally-generated image (such as a Philips® 3D-RA™ or XperCT™) or a pre-procedural image (e.g., magnetic resonance (MR) or computed tomography (CT)). The image is intended to closely correspond to the patient's anatomy for the duration of time that it is used as an overlay. However, it is widely known that a stiff instrument can significantly deform a vessel's shape by pressing against its walls.

Although interventional devices are inside the patient's vessels, their trajectories on a fluoroscopic image may lie, in part, outside the static 3D overlay due to deformation of the real anatomy by the instruments. As a result, pre-procedural image overlays may not be accurate or clinically useful for guiding an interventional device into or through narrow lumens (e.g., a small vascular sidebranch).

Multiple technologies for 3D localization and sensing along an interventional device include the following. Electromagnetic (EM) localization where single-point EM sensors can be used to accurately localize points at intervals along an interventional device. By interpolating between these points, the 3D device shape can be determined. Fiber-optic shape sensing based on scattering from Fiber Bragg Gratings (FBG) or Rayleigh scattering is another approach that permits the entire device shape in three dimensions to be determined. X-ray-based 3D device shape determination may also be used to interrogate the 3D shape of an interventional device from x-ray alone, using a combination of known device-based x-ray markers and x-ray system geometry to estimate location and configuration of the interventional device. Given any particular imaging system geometry, the shapes of these markers on an x-ray image could vary depending on their 3D orientations, which depend, in turn, on the interventional device shapes. Therefore, x-ray markers may be used to approximate the 3D device shape. Characteristics of the device shape may also be determined with other sensing schemes occurring simultaneously with fluoroscopy, such as with ultrasound (conventional imaging or ultrasound time-of-flight localization of beacons embedded within the device), photoacoustics, impedance-based localization, etc.

Document US2005/0182319 A1 discloses a system for registering, verifying, dynamically referencing, and navigating an anatomical region of interest of a patient. The anatomical region of interest is imaged using an imaging via an x-ray device. A tracked registration device is removably inserted in a conduit within the anatomical region and the position of the registration device is sampled by a tracking device as the registration device is moved within the anatomical region through the catheter. The sampled position data is registered to the image data to register the path of the conduit to the anatomical region of interest. The same device is used to dynamically reference the movements affecting the anatomical region and modify the registration in real time.

The invention is defined in the independent claims. Preferred embodiments are defined in the dependent claims.

In accordance with the present principles, a system includes a medical imaging system configured to generate images of an interventional procedure. An overlay generator is configured to generate an overlay image on the images of the interventional procedure. An interventional device tracking system is configured to track a 3D position, orientation and shape of the interventional device during the procedure, where the overlay image is dynamically updated in response to deformations caused to an organ of interest by the interventional device during the procedure.

A method for a medical procedure embedded into a computer program product includes generating images of an interventional procedure; generating an overlay image on the images of the interventional procedure; tracking a position, orientation and shape of the interventional device during the procedure; dynamically updating the overlay image in response to deformations caused to an organ of interest by the interventional device during the procedure.

Another method for a medical procedure embedded into a computer program product includes generating images of an interventional procedure; generating an overlay image on the images of the interventional procedure; tracking a position, orientation and shape of the interventional device during the procedure; checking whether the interventional device remains within a boundary of the overlay image; if the interventional device is not fully enclosed in the boundary, determining a deformation of the organ that will permit the interventional device to remain within the boundary; and dynamically updating the overlay image in accordance with the deformation.

These and other objects, features and advantages of the present disclosure will become apparent from the following detailed description of illustrative embodiments thereof, which is to be read in connection with the accompanying drawings.

The invention is defined in the independent claims 1 and 9.

This disclosure will present in detail the following description of preferred embodiments with reference to the following figures wherein:
FIG. 1 is a block/flow diagram showing a system/method for updating an overlay image in accordance with the present principles;
FIG. 2 is a flow diagram showing a method for performing a procedure with updated overlay images in accordance with one illustrative embodiment;
FIG. 3 is a flow diagram showing a method for updating an overlay image using models in accordance with another illustrative embodiment; and
FIG. 4 is a flow diagram showing a method for updating an overlay image in accordance with another illustrative embodiment.

The present disclosure describes three-dimensional (3D) image overlay systems and methods. The present embodiments improve accuracy of 3D image overlays on live fluoroscopy images for interventional procedures by non-rigid-body warping of the 3D image of an organ based on the 3D shape of the interventional device within that organ. This technique could be applied in any (e.g., minimally-invasive) interventional vascular, luminal or interstitial procedure where highly precise device placement is needed and/or the tissue morphology (e.g., the vessel in a vascular procedure) is significantly affected by inserting a rigid or semi-rigid device (e.g., abdominal or thoracic aorta stent-grafting, carotid artery stenting, Uterine fibroid embolizations (UFEs), Transjugular Intrahepatic Portosystemic Shunt (TIPS), Transarterial Chemoembolization (TACE) procedures), etc. The present embodiments may be employed in any interventional vascular procedures, e.g., where highly precise device-placement is needed and/or the vessel morphology is significantly affected by inserting a comparatively stiff device.

It also should be understood that the present invention will be described in terms of medical instruments; however, the teachings of the present invention are much broader and are applicable to any instruments employed in tracking or analyzing complex biological or mechanical systems. In particular, the present principles are applicable to internal tracking procedures of biological systems, procedures in all areas of the body such as the lungs, gastro-intestinal tract, excretory organs, blood vessels, etc. The elements depicted in the FIGS. may be implemented in various combinations of hardware and software and provide functions which may be combined in a single element or multiple elements.

The functions of the various elements shown in the FIGS. can be provided through the use of dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), non-volatile storage, etc.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (i.e., any elements developed that perform the same function, regardless of structure). Thus, for example, it will be appreciated by those skilled in the art that the block diagrams presented herein represent conceptual views of illustrative system components and/or circuitry embodying the principles of the invention. Similarly, it will be appreciated that any flow charts, flow diagrams and the like represent various processes which may be substantially represented in computer readable storage media and so executed by a computer or processor, whether or not such computer or processor is explicitly shown.

Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W) and DVD.

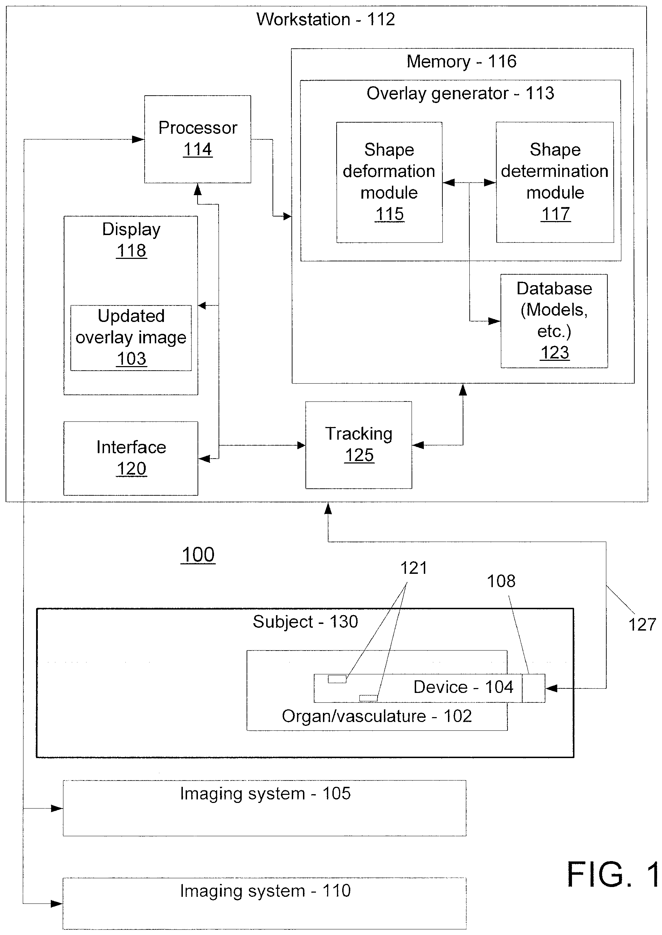

Referring now to the drawings in which like numerals represent the same or similar elements and initially to FIG. 1, a system 100 for maintaining registration between a fluoroscopy image and an overlay is illustratively depicted. The system 100 improves the accuracy of a 3D image overlay on a live imaging (e.g., fluoroscopy) system or platform 105 for vascular interventional procedures. In one embodiment, imaging system 105 includes a fluoroscopy system which may be employed without the use of contrast dyes as a result of the dynamic overlay in accordance with the present principles.

The system 100 includes an interventional device 104 intended for insertion into vascular structures. The interventional device 104 may include a catheter, a probe, a diagnostic sensor, a guidewire, a therapy delivering element, a needle for biopsy or therapy delivery (e.g. injection of biologics, ablative, or embolic material), a cutting device, a shaping device, a prosthetic support device, an endoscope, a robot, an electrode, a filter device, a balloon device or any other rigid, semi-rigid or fully flexible instrument. The interventional device 104 may be coupled to a workstation or other console 112 by a cable 127 or other connection. A procedure is supervised and/or managed from the workstation or console 112. Workstation 112 preferably includes one or more processors 114 and memory 116 for storing programs and applications.

Memory 116 includes an overlay generator 113. Overlay generator 113 may include a shape deformation module 115 configured to interpret feedback signals from device 104 and determine a new shape for an organ or vasculature 102 affected by the device 104 during a procedure. The shape deformation module 115 accepts as input a set of parameters describing how an image space is deformed, and produces as output a deformed 3D anatomical image of the vasculature 102. Overlay generator 113 also includes or receives input from a shape determination module 117 for determining a 3D shape of the interventional device(s) 104 and a position(s) of the device 104 in image space. The module 117 may also measure or track other parameters such as pressure, strain, shear, or proximity/contact sensors 121 on the device 104 to provide additional feedback measurements.

Modules 115 and 117 work together to provide updated 3D overlay images which are consistent with a shape and position of the device 104 as it is moved into or through a vasculature. There is a data connection between modules 115 and 117 that permits an estimate of the 3D shape(s) of the interventional device(s) 104 to be used to determine the set of parameters provided as input to the shape deformation module 115. The parameters are chosen so that the vascular structures in a deformed 3D anatomical image 103 are consistent with the estimated shape of the interventional device 104.

A database 123 is stored in memory or is accessible over a network and includes historic data, models, and/or finite element representations of organs and their deformation response to particular instruments for particular procedures. The database 123 may be employed by either or both modules 115 and 117 to update the 3D images for a dynamic overlay.

In one embodiment, pressure or other sensors 121 are mounted on the device 104 so that pressure or other measurements can be taken and recorded. Other instrumentality 108 such as, e.g., optical fiber sensing (Fiber Bragg gratings (FBG), Rayleigh scattering optical fiber, etc.), EM tracking or other device localization / configuration determining capability may be employed to further determine a position and shape of the device 104. Since the position and shape of the device 104 is known, the pressure measurements, etc. provide additional information about the device 104 interacting with the vasculature 102. A determination of tissue that is displaced as a result of the device 104 may be computed by shape deformation module 115. Shape deformation module 115 is configured to use any other feedback from device 104 to reconstruct 3D deformations, deflections and other changes associated with a medical device or instrument 104 and/or its surrounding region. Device tracking 125 is employed to estimate non-rigid body morphing of a 3D image (from pre-procedural imaging or intra-procedural 3D treatment, ultrasound, etc.). The morphing is computed such that the morphed 3D image 103 more accurately reflects a real-time vascular anatomy.

Workstation 112 may include a display 118 for viewing internal images of a subject 130. In addition to the fluoroscopy or other real-time imaging platform 105, an imaging system 110 may optionally be provided. Imaging system 110 may include a magnetic resonance imaging (MRI) system, a computed tomography (CT) system, an ultrasound system, a nuclear imaging system or any other system configured to generate 3D images of the subject 130. The system 100 may or may not include such an imaging system 110 as images may be taken *a priori* and sent to the workstation 112 over a network or transferred to the workstation via a memory storage device.

Display or displays 118 may be employed to render fluoroscopy or other real-time images and 3D overlays (from images previously taken of the subject 130). The 3D overlay images include the vasculature through which the interventional device(s) 104 is/are guided. Display 118 also permits a user to interact with the workstation 112 and its components and functions, or any other element within the system 100. This is further facilitated by an interface 120 which may include a keyboard, mouse, a joystick, a haptic device, or any other peripheral or control to permit user feedback from and interaction with the workstation 112.

System 100 may include a 3D tracking technology 125, such as EM tracking, optical shape sensing or a similar 3D position or orientation sensing system which may be integrated with the workstation 112 or be a separate system. An EM tracking system 125 includes an EM sensing module used to interpret EM signals generated by the medical device 104 during a procedure. The medical device 104 may include one or more EM tracking sensors, which may be mounted on the device 104. The medical device 104 may also include a fiber optic shape sensing device (125) which provides optical readings that are reconstructed into information about device location, orientation, and shape.

Workstation 112 may include an optical interrogation unit or module (125), which is employed to transmit light and detect light returning from all fibers if optical fiber sensing is employed. This permits the determination of strains or other parameters, which will be used to interpret the shape, orientation, or other characteristics, sensed by the interventional device 104. The light signals will be employed as feedback to understand the device 104 to tissue interaction in the subject 130. The shape determination module 117 and the shape deformation module 115 are employed to compute a new overlay image that accounts for deformations due to device - tissue interactions. This improves the accuracy of the overlay 103 making the image closer to an actual organ shape during a procedure.

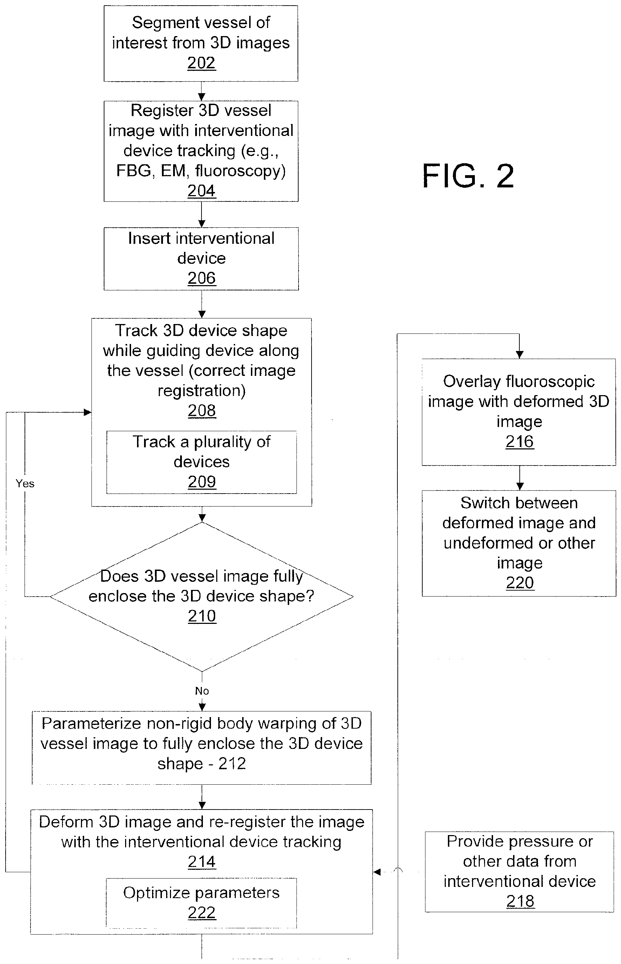

Referring to FIG. 2, a block/flow diagram is shown for updating a three-dimensional overlay in accordance with tissue morphing as a result of a medical device for one illustrative embodiment. In block 202, 3D vessel imaging and segmentation of vessels of interest is performed. Image acquisition and segmentation of vessels from such images may be performed using known techniques. In block 204, a 3D shape of the interventional device is determined, for example, by EM localization of one or more points along the interventional device, optical shape sensing (using optical interrogation of fiber strains and 3D reconstruction of strain fields to track the continuous device shape in three dimensions) or x-ray-based 3D device shape sensing (e.g., using 3D markers at intervals along the device). The registration of 3D images to tracking technologies may employ known techniques. In block 206, a clinician guides an interventional device into or through a vessel of interest.

In block 208, the 3D shape of the device is tracked while guiding the device along a vasculature. The 3D shape of the interventional device determined using the tracking technology should lie within the vessel boundaries displayed in the 3D vessel image. This is checked in block 210.

In block 209, in one embodiment, the shapes of multiple interventional devices may be tracked and a parameterization method in block 212 would involve determining the anatomy with a 3D vessel shape that optimally encompasses the shape of all the 3D devices, based on both the available measurements and the accumulated prior knowledge available about the procedure and device(s) of interest.

In block 210, the device should be physically contained by walls of the vessel. Therefore, if the 3D vessel image is a true representation of the real-time patient anatomy, the device tracking technology and 3D imaging space are accurately co-registered, and the device remains within the vasculature.

If this is not the case, this could be due to patient or table movement. This causes a translation of the 3D image and device tracking space, which needs to be accounted for. Another misregistration may be due to respiratory or cardiac movement. This may cause a cyclical movement of vessels that are within the patient's thorax or proximal to the diaphragm. There are *many* vessels whose morphologies are not affected by the respiratory or cardiac cycles (e.g. abdominal aorta, limb arteries, neuro vessels etc.). These cases may be accounted for in rigid body movement and/or cyclic compensation algorithms.

Another misregistration may be from the device causing vessel deformation. Non-rigid-body warping of the 3D image is needed to update the 3D image so that it better reflects the real-time anatomy of the patient in accordance with the present principles.

In block 212, the non-rigid-body deformation of the 3D image is parameterized so that it more accurately reflects the patient's real-time anatomy. This may be achieved in multiple ways.

In block 214, one example of non-rigid-body warping is to assume that the 3D anatomy enclosing the vessel has deformed so that the vessel can continue to enclose the tracked 3D shape of the interventional device, and that the vessel diameter/length remains constant. The parameterization method then involves determining the anatomy with a 3D vessel shape that encompasses the 3D device shape. After the 3D image is deformed, it is reregistered with the device by returning to block 208, if needed.

More advanced examples may permit for the deformation of both the 3D anatomy enclosing the vessel and the vessel diameter/length. In these examples, it would be useful to include vessel characteristics such as longitudinal and radial vessel elasticities and an estimate of vessel deformability in that anatomical area (which is affected, for example, by the degree of vessel calcification - seen on CT and fluoroscopy - and the number of spinal arteries). Characteristics of the 3D image would therefore be useful to determine what types of deformations should be applied.

In block 222, in one embodiment, optimal parameters utilized for deformation of the 3D image may optionally be determined by taking into account parameters obtained at previous time points. With assumptions about continuity of the anatomical deformations in time, the space of parameters that is considered could be significantly constrained, which could in turn lead to faster processing times and/or predictable deformations. Parameter optimization may rely on other data as well.

For example, in block 218, one embodiment provides pressure or other parametric sensing along the interventional device (either at distinct points or continuously along a segment as could be provided by FBGs, for example). This provides new information on which points/segments of the interventional device are in contact with the vessel wall and which points/segments are floating freely within the lumen of the vessel. This also provides valuable information on the true vessel shape that can be employed to improve the non-rigid-body parameterization process.

In block 216, the fluoroscopic or real-time image is overlaid with the deformed 3D image.

In block 220, in one embodiment, a user/clinician is provided with a capability of switching back and forth between the warped 3D image and a non-warped 3D image via an interface (120, FIG. 1). By comparing the two images, the clinician can assess how much pressure is being applied to the vessel walls (and whether this pressure could be dangerous and lead to potential vessel rupture). Elastographic measurements could also be obtained automatically by analyzing the deformations obtained for the 3D images.

The process is updated throughout a procedure. This provides an updated and accurate overlay during the procedure.

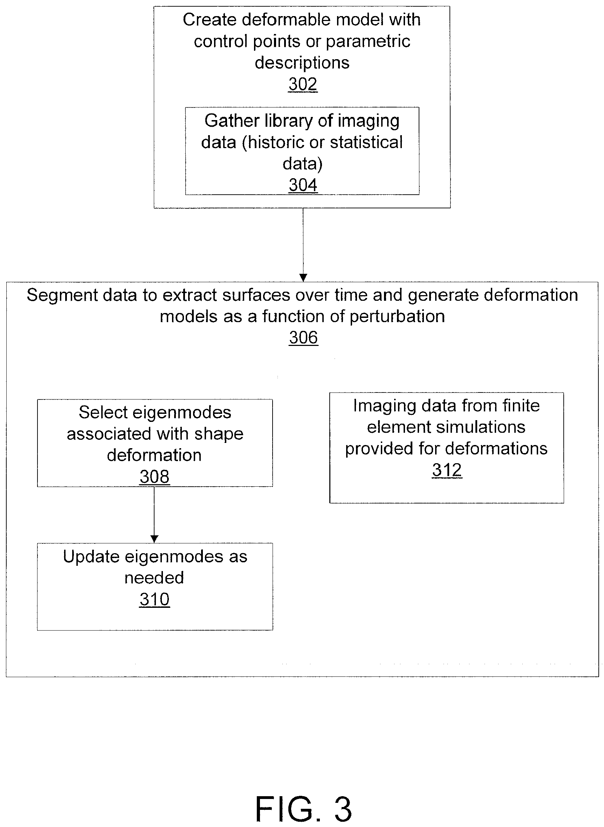

Referring to FIG. 3, another embodiment employs statistical or historic data to deform vessel images in accordance with the present principles. In block 302, a derivation of an anatomically-realistic deformable model is created with a minimal set of control points or parametric descriptions. A sparse set of tracked landmarks (localized in 3D by EM, impedance, acoustic, optical sensing, etc.) is available from the device and delivery assembly (e.g., a catheter or sheath) for adapting the rigid-body pose and deformation of the model. In block 304, one way of building this model includes gathering a library of imaging data acquired during an intervention (e.g., historic or statistical data). The data shows dynamic behavior of the anatomy as the instrumentation is moved within it.

In block 306, segmentation of the data can be performed to extract 3D surfaces evolving over time, and deformation models of the surface shape change as a function of perturbation are generated. In particular, a principal component analysis (or similar analytical methods for decomposition of shapes into component modes) may be employed to determine eigenmodes of shape deformation and associated eigenvalues which reflect the relative importance of each shape eigenvector. In block 308, a subset of eigenmodes associated with the largest eigenvalues can be selected to minimize the parameter space associated with shape deformation, while ensuring that the dominant deformation behaviors are still captured within the parametric model. Adaptation of the library-derived models to a particular patient anatomy (from 3D preprocedural data segmentation) and particular set of tracked feature points on the device can then occur by estimating the eigenmode coefficients/weighting values which minimize a distance metric computed between the model and observed measurements.

In block 310, the eigenmodes are updated as needed during the procedure to ensure that the model follows closely the deflection of the tissue as a result of the medical instrument. In this way, a more clinically meaningful display of tissue response may be projected in an overlay image, in particular, during a fluoroscopically tracked procedure. Other information about deformation functions of the anatomy of interest may be derived from vector velocity fields of pre-procedural phase-contrast MR imaging or tissue speckle tracking with ultrasound imaging. These other sources of measurement information can augment the prior knowledge available from libraries of segmented 3D surface models.

In addition to or instead of computing eigenmodes, a library of imaging data from actual studies may be substituted by a library of deformations that can be derived from finite element simulations of the anatomy deforming under instrument manipulation, in block 312. A host of potential libraries and training datasets for computing appropriate models for a range of different clinical interventions and instrumentation can be generated to broaden the scope of applicability.

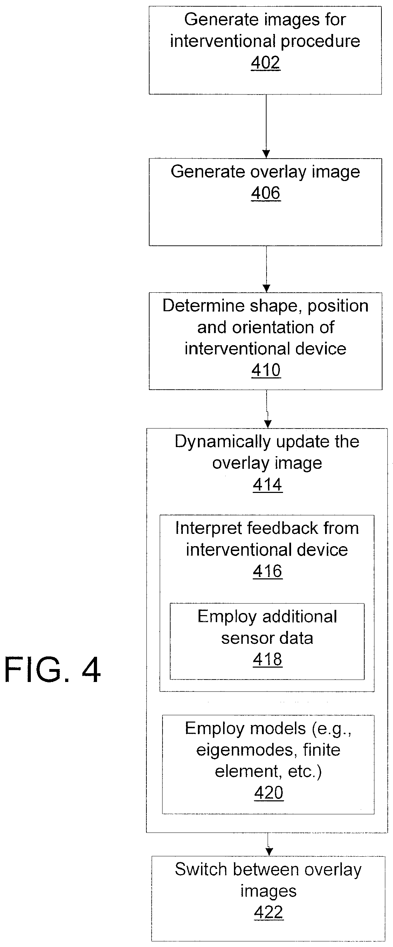

Referring to FIG. 4, a medical procedure is illustratively shown in accordance with another embodiment. In block 402, images of an interventional procedure are generated. These images are preferably real-time images generated using fluoroscopic imaging. In block 406, an overlay image is generated on the images of the interventional procedure. The overlay images include three-dimensional anatomical images of a subject taken by, e.g., computed tomography, magnetic resonance imaging or other imaging methods.

In block 410, a position, orientation and shape of the interventional device (in 3D) are tracked during the procedure. The tracking may include using one or more of electromagnetic tracking, optical sensing, fluoroscopy marker tracking, etc.

In block 414, the overlay image is dynamically updated in response to 3D deformations caused to an organ of interest by the interventional device during the procedure. Updating the overlay image preferably includes interpreting feedback signals from the interventional device and determining a new shape for the organ affected by the interventional device in block 416. In block 418, the interventional device includes pressure sensors or other sensors, and measurements (e.g., pressures) are employed to determine a deformation response of the organ.

In block 420, models of deformation responses of the organ may be stored and employed to update the overlay image of the organ. The models may generated by computing eigenmodes of tissue response, generated in accordance with finite element simulations or employ historic or statistical data to reproduce or predict organ movement. In block 422, a capability is provided to enable switching between the overlay image and an updated overlay image during the interventional procedure. In this way, the updated overlay can be compared with a previous or original overlay image.

In interpreting the appended claims, it should be understood that:
a) the word "comprising" does not exclude the presence of other elements or acts than those listed in a given claim;
b) the word " a" or "an" preceding an element does not exclude the presence of a plurality of such elements;
c) any reference signs in the claims do not limit their scope;
d) several "means" may be represented by the same item or hardware or software implemented structure or function; and
e) no specific sequence of acts is intended to be required unless specifically indicated.

Having described preferred embodiments for systems and methods for non-rigid-body morphing of vessel image using intravascular device shape (which are intended to be illustrative and not limiting), it is noted that modifications and variations can be made by persons skilled in the art in the scope of the claims. It is therefore to be understood that changes may be made in the particular embodiments of the disclosure disclosed which are within the scope of the embodiments disclosed herein as outlined by the appended claims. Having thus described the details and particularity required by the patent laws, what is claimed and desired protected by Letters Patent is set forth in the appended claims.

## Claims

1. A medical system, comprising:
a medical imaging system (105) configured to generate images of an interventional procedure;
an overlay generator (113) configured to generate an overlay image on the images of the interventional procedure, wherein the overlay image includes a three-dimensional (3D) anatomical image; and
an interventional device tracking system (108, 125) configured to dynamically track a 3D position, orientation and shape of an interventional device (104) during the procedure;
wherein the overlay image is dynamically updated in response to deformations caused to an organ of interest by the interventional device during the procedure, said deformations being determined based on sensing of pressure, strain, shear or proximity/contact along said interventional device.

2. The medical system as recited in claim 1, wherein the overlay generator (113) includes a shape deformation module (115) configured to interpret feedback signals from the interventional device and determine a new shape for the organ affected by the interventional device.

3. The medical system as recited in claim 1, wherein the overlay generator (113) includes a shape determination module (117) for determining the position, the orientation and shape of the interventional device in image space.

4. The medical system as recited in claim 1, wherein the interventional device (104) includes at least one of pressure, strain, shear, or proximity/contact sensors and sensor measurements are employed to determine a deformation response of the organ.

5. The medical system as recited in claim 1, further comprising a database (123) configured to store models of deformation responses of the organ which are employed by the overlay module to update the overlay image of the organ.

6. The medical system as recited in claim 5, wherein the database (123) stores at least one of eigenmodes of tissue response and finite element simulations.

7. The medical system as recited in claim 1, wherein the interventional device tracking system (125) includes at least one of electromagnetic tracking, optical sensing, or fluoroscopy marker tracking.

8. The medical system as recited in claim 1, wherein the overlay images (103) including 3D anatomical images of a subject are taken by at least one of computed tomography, magnetic resonance imaging, ultrasound or nuclear imaging.

9. A computer program product comprising instructions which, when the program is executed by a computer, cause the computer to carry out steps of:
generating (402) images of an interventional procedure;
generating (406) an overlay image on the images of the interventional procedure, wherein the overlay image includes a 3D anatomical image;
tracking (410) a 3D position, orientation and shape of the interventional device during the procedure;
dynamically updating (414) the overlay image in response to deformations caused to an organ of interest by the interventional device during the procedure, said deformations being determined based on sensing of pressure, strain, shear or proximity/contact along said interventional device.

10. The computer program product as recited in claim 9, wherein updating (414) the overlay image includes interpreting (416) feedback signals from the interventional device and determining a new shape for the organ affected by the interventional device.

11. The computer program product as recited in claim 9, wherein the interventional device includes at least one of pressure, strain, shear, or proximity/contact sensors and sensor measurements are employed (418) to determine a deformation response of the organ.

12. The computer program product as recited in claim 9, further causing the computer to carry out steps of storing (420) models of deformation responses of the organ which are employed to update the overlay image of the organ.

13. The method as recited in claim 12, wherein the models are generated by computing eigenmodes of tissue response.

14. The computer program product as recited in claim 12, wherein the models are generated in accordance with finite element simulations.

15. The computer program product as recited in claim 9, further causing the computer to carry out steps of:
checking (210) whether the interventional device remains within a boundary of the overlay image; and
if the interventional device is not fully enclosed in the boundary, determining (212) a deformation of the organ that will permit the interventional device to remain within the boundary; and wherein
dynamically updating (414) includes dynamically updating (214) the overlay image in accordance with the deformation.

## Patentansprüche

1. Medizinisches System, das Folgendes umfasst:
ein medizinisches Bildgebungssystem (105), das konfiguriert ist, um Bilder einer Eingriffsvorgehensweise zu erzeugen;
einen Overlay-Generator (113), der konfiguriert ist, um ein Overlay-Bild auf den Bildern der Eingriffsvorgehensweise zu erzeugen, wobei das Overlay-Bild ein dreidimensionales (3D) anatomisches Bild beinhaltet; und
ein Eingriffsvorrichtungsverfolgungssystem (108, 125), das konfiguriert ist, um dynamisch eine 3D-Position, eine Ausrichtung und eine Form einer Eingriffsvorrichtung (104) während der Vorgehensweise zu verfolgen;
wobei das Overlay-Bild dynamisch als Reaktion auf Verformungen, die an einem Organ von Interesse von der Eingriffsvorrichtung während der Vorgehensweise verursacht werden, aktualisiert wird, wobei die Verformungen basierend auf Erfassen von Druck, Belastung, Scheren oder Nähe/Kontakt entlang der Eingriffsvorrichtung bestimmt werden.

2. Medizinisches System nach Anspruch 1, wobei der Overlay-Generator (113) ein Formverformungsmodul (115) beinhaltet, das konfiguriert ist, um Feedbacksignale von der Eingriffsvorrichtung auszulegen und eine neue Form für das Organ, das von der Eingriffsvorrichtung beeinflusst wird, zu bestimmen.

3. Medizinisches System nach Anspruch 1, wobei der Overlay-Generator (113) ein Formbestimmungsmodul (117) zum Bestimmen der Position, der Ausrichtung und der Form der Eingriffsvorrichtung in einem Bildraum beinhaltet.

4. Medizinisches System nach Anspruch 1, wobei die Eingriffsvorrichtung (104) mindestens einen eines Druck-, Belastungs-, Scher- oder Nähe-/Kontaktsensors beinhaltet, und Sensormessungen eingesetzt werden, um eine Verformungsreaktion des Organs zu bestimmen.

5. Medizinisches System nach Anspruch 1, das weiter eine Datenbank (123) umfasst, die konfiguriert ist, um Modelle von Verformungsreaktionen des Organs zu speichern, die von dem Overlay-Modul eingesetzt werden, um das Overlay-Bild des Organs zu aktualisieren.

6. Medizinisches System nach Anspruch 5, wobei die Datenbank (123) mindestens einen von Gewebereaktionseigenmodi und Finite-Element-Simulationen speichert.

7. Medizinisches System nach Anspruch 1, wobei das Eingriffsvorrichtungsverfolgungssystem (125) mindestens eine einer elektromagnetischen Verfolgung, optischer Erfassung oder Fluoroskopiemarkerverfolgung beinhaltet.

8. Medizinisches System nach Anspruch 1, wobei die Overlay-Bilder (103), die anatomische 3D-Bilder eines Subjekts beinhalten, durch mindestens eines von Computertomografie, Magnetresonanzbildgebung, Ultraschall- oder Nuklearbildgebung aufgenommen werden.

9. Computerprogrammprodukt, das Anweisungen umfasst, die, wenn das Programm von einem Computer ausgeführt wird, den Computer veranlassen, folgende Schritte auszuführen:
Erzeugen (402) von Bildern einer Eingriffsvorgehensweise;
Erzeugen (406) eines Overlay-Bilds auf den Bildern der Eingriffsvorgehensweise, wobei das Overlay-Bild ein anatomisches 3D-Bild beinhaltet;
Verfolgen (410) einer 3D-Position, Ausrichtung und Form der Eingriffsvorrichtung während der Vorgehensweise;
dynamisches Aktualisieren (414) des Overlay-Bilds als Reaktion auf Verformungen, die an einem Organ von Interesse von der Eingriffsvorrichtung während der Vorgehensweise verursacht werden, wobei die Verformungen basierend auf Erfassen von Druck, Belastung, Scheren oder Nähe/Kontakt entlang der Eingriffsvorrichtung bestimmt werden.

10. Computerprogrammprodukt nach Anspruch 9, wobei das Aktualisieren (414) des Overlay-Bilds das Auslegen (416) von Feedbacksignalen von der Eingriffsvorrichtung und das Bestimmen einer neuen Form für das Organ, das von der Eingriffsvorrichtung beeinflusst wird, beinhaltet.

11. Computerprogrammprodukt nach Anspruch 9, wobei die Eingriffsvorrichtung mindestens einen eines Druck-, Belastungs-, Scher- oder Nähe-/Kontaktsensors beinhaltet, und Sensormessungen eingesetzt werden (418), um eine Verformungsreaktion des Organs zu bestimmen.

12. Computerprogrammprodukt nach Anspruch 9, das weiter den Computer veranlasst, Schritte zum Speichern (420) von Modellen von Verformungsreaktionen des Organs auszuführen, die eingesetzt werden, um das Overlay-Bild des Organs zu aktualisieren.

13. Verfahren nach Anspruch 12, wobei die Modelle durch Berechnen von Gewebereaktionseigenmodi erzeugt werden.

14. Computerprogrammprodukt nach Anspruch 12, wobei die Modelle in Übereinstimmung mit Finite-Element-Simulationen erzeugt werden.

15. Computerprogrammprodukt nach Anspruch 9, das weiter den Computer veranlasst, folgende Schritte auszuführen:
Prüfen (210), ob die Eingriffsvorrichtung innerhalb einer Grenze des Overlay-Bilds bleibt; und
falls die Eingriffsvorrichtung nicht vollständig von der Grenze umschlossen ist,
Bestimmen (212) einer Verformung des Organs, die es der Eingriffsvorrichtung erlaubt, innerhalb der Grenze zu bleiben; und wobei
das dynamische Aktualisieren (414) das dynamische Aktualisieren (214) des Overlay-Bilds in Übereinstimmung mit der Verformung beinhaltet.

## Revendications

1. Système médical comprenant :
un système d'imagerie médicale (105) configuré pour générer des images d'une procédure d'intervention ;
un générateur de recouvrement (113) pour configurer pour générer une image de recouvrement sur les image de la procédure d'intervention, dans lequel l'image de recouvrement comprend une image anatomique à trois dimensions (3D) ; et
un système de suivi d'intervention (108, 125) configuré pour suivre de manière dynamique une position 3D, une orientation et une forme du dispositif d'intervention (104) au cours de la procédure ;
dans lequel l'image de recouvrement est mise à jour de manière dynamique en réponse à des déformations provoquées à un organe d'intérêt par le dispositif d'intervention au cours de la procédure, lesdites déformations étant déterminées sur la base d'une détection de pression, de tension, de cisaillement ou de proximité/contact le long dudit dispositif d'intervention.

2. Système médical selon la revendication 1, dans lequel le générateur de recouvrement (113) comprend un module de déformation (115) configuré pour interpréter des signaux de rétroaction venant du dispositif d'intervention et déterminer une nouvelle forme pour l'organe affecté par le dispositif d'intervention.

3. Système médical selon la revendication 1, dans lequel le générateur de recouvrement (113) comprend un module de détermination de forme (117) pour déterminer la position, l'orientation et la forme du dispositif d'intervention dans l'espace d'image.

4. Système médical selon la revendication 1, dans lequel le dispositif d'intervention (104) comprend au moins un de capteurs de pression, de tension, de cisaillement ou de proximité/contact et des mesures des capteurs sont utilisées pour déterminer une réponse à la déformation de l'organe.

5. Système médical selon la revendication 1, comprenant en outre une base de données (123) configurée pour stocker des modèles de réponses à la déformation de l'organe qui sont utilisées par le module de recouvrement pour mettre à jour l'image de recouvrement de l'organe.

6. Système médical selon la revendication 5, dans lequel la base de données (123) stocke au moins un des modes propres de réponse des tissus et de stimulations d'éléments finis.

7. Système médical selon la revendication 1, dans lequel le système de suivi (125) du dispositif d'intervention comprend au moins l'un parmi un suivi électromagnétique, un suivi optique ou un suivi de marqueur de fluoroscopie.

8. Système médical selon la revendication 1, dans lequel les images de recouvrement (103) comprenant des images anatomiques 3D d'un sujet sont prises par au moins l'une parmi une tomographie à calculateur, une imagerie par résonance magnétique, une imagerie ultrasonore ou nucléaire.

9. Produit de programme d'ordinateur comprenant des instructions qui, lorsque le programme est exécuté par un ordinateur, amènent l'ordinateur à effectuer les étapes consistant à :
générer (402) des images d'une procédure d'intervention ;
générer (406) une image de recouvrement sur les images de la procédure d'intervention, dans lequel l'image de recouvrement comprend une image anatomique 3D ;
suivre (410) une position 3D, une orientation et une forme du dispositif d'intervention au cours de la procédure ;
mettre à jour de manière dynamique (414) l'image de recouvrement en réponse à des déformations provoquées à un organe d'intérêt par le dispositif d'intervention au cours de la procédure, lesdites déformations étant déterminées sur la base de la détection de pression, tension, cisaillement ou proximité/contact le long dudit dispositif d'intervention.

10. Produit de programme d'ordinateur selon la revendication 9, dans lequel la mise à jour (414) de l'image de recouvrement comprend l'interprétation (416) de signaux de rétroaction venant du dispositif d'intervention et la détermination d'une nouvelle forme pour l'organe affecté par le dispositif d'intervention.

11. Produit de programme d'ordinateur selon la revendication 9, dans lequel le dispositif d'intervention comprend au moins l'un(e) de capteurs de pression, de tension, de cisaillement ou de proximité/contact et des mesures des capteurs sont employées (418) pour déterminer une réponse à la déformation de l'organe.

12. Produit de programme d'ordinateur selon la revendication 9, amenant en outre l'ordinateur à effectuer les étapes de stockage (420) de modèles de réponses à la déformation de l'organe qui sont employés pour mettre à jour l'image de recouvrement de l'organe.

13. Procédé selon la revendication 12, dans lequel les modèles sont générés en calculant des modes propres de réponse des tissus.

14. Produit de programme d'ordinateur selon la revendication 12, dans lequel les modèles sont générés conformément à des simulations d'éléments finis.

15. Produit de programme d'ordinateur selon la revendication 9, amenant en outre l'ordinateur à effectuer les étapes consistant à :
vérifier (210) si le dispositif d'intervention reste ou non dans une limite de l'image de recouvrement ; et,
si le dispositif d'intervention n'est pas pleinement enserré dans la limite, déterminer (212) une déformation de l'organe qui permettra au dispositif d'intervention de rester dans la limite ; et dans lequel :
la mise à jour dynamique (414) comprend une mise à jour dynamique (214) de l'image de recouvrement conformément à la déformation.
